Europäisches Patentamt

European Patent Office

Office européen des brevets

(11) Veröffentlichungsnummer: **0 093 259**
**B1**

(12) **EUROPÄISCHE PATENTSCHRIFT**

(45) Veröffentlichungstag der Patentschrift:
**03.06.87**

(21) Anmeldenummer: **83102812.1**

(22) Anmeldetag: **22.03.83**

(51) Int. Cl.⁴: **A 61 M 1/14, A 61 M 1/00**

(54) Vorrichtung zur Bilanzierung des Flüssigkeitsaustausches bei Hämofiltrationen.

(30) Priorität: **10.04.82 DE 3213390**

(43) Veröffentlichungstag der Anmeldung:
**09.11.83 Patentblatt 83/45**

(45) Bekanntmachung des Hinweises auf die Patenterteilung:
**03.06.87 Patentblatt 87/23**

(84) Benannte Vertragsstaaten:
**AT BE CH DE FR GB IT LI LU NL SE**

(56) Entgegenhaltungen:
**DE - A - 1 566 680**
**DE - A - 2 629 717**
**US - A - 3 242 924**
**US - A - 3 425 415**

(73) Patentinhaber: **Schurek, H.J., Dr.med., Sperlingsfeld 9,**
**D-3000 Hannover 61 (DE)**
Patentinhaber: **Biela, Dieter, Wilhelm-Blum-Strasse 19,**
**D-3000 Hannover 91 (DE)**

(72) Erfinder: **Schurek, H.J., Dr.med., Sperlingsfeld 9,**
**D-3000 Hannover 61 (DE)**
Erfinder: **Biela, Dieter, Wilhelm-Blum-Strasse 19,**
**D-3000 Hannover 91 (DE)**

(74) Vertreter: **Arendt, Helmut, Dipl.-Ing., Hubertusstrasse 2,**
**D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft eine Vorrichtung zur kontinuierlichen Bilanzierung von Filtrat und Substitutionslösung bei therapeutischen Verfahren zur Eliminierung von Schadstoffen aus dem menschlichen Organismus, insbesondere zur Bilanzierung bei spontanen arterio-venösen Blutfiltrationen mit einer mechanischen Waage, deren Lastseite durch die Summe der Massen des Filtrats und der Substitutionslösung belastbar ist und mit einem mechanisch mit der Waage verbundenen Drossel- und Absperrorgan zur Steuerung der Zufuhr der Substitutionslösung.

Bei akutem oder chronischem Nierenversagen werden überschüssiges Wasser und Stoffwechselschlacken des Körpers bis heute überwiegend mit der Hämodialyse entfernt. Bei der Hämodialyse ist es notwendig, technisch aufwendige und elektronisch monitorisierte Geräte unter hohen Kosten einzusetzen. Die Geräte sind auf einen Stromanschluss und einen Anschluss an Reinstwasser (entionisiert) angewiesen. Darüberhinaus müssen die Geräte durch Techniker regelmässig gewartet werden und können nur durch spezialisierte Pflegekräfte und Ärzte bedient werden. Eine Standardbehandlung mit der künstlichen Niere besteht in einer 3–5stündigen Hämodialyse während einer Woche. Da überschüssige Flüssigkeit und Schlaken in den kurzen Behandlungszyklen abrupt entzogen werden, sind Kreislaufstörungen mit Blutdruckabfall eine häufige Komplikation. Das gleiche gilt im wesentlichen auch für die maschinelle Hämofiltration, wenn auch quantitative Unterschiede bestehen.

Ein kürzlich entwickeltes Behandlungsverfahren bei akutem Nierenversagen ist die kontinuierliche arterio-venöse Hämofiltration, die besonders bei Intensivpflegepatienten, die immobilisiert und beatmet werden, eingesetzt wird. Durch diese Behandlungstechnik können Nachteile der konventionellen Therapie vermieden und Kosten eingespart werden.

Bei diesem Verfahren wird ein kleines Kapillarhämofilter an einen arteriellen und venösen Gefässzugang des Patienten angeschlossen. Die Filterkapillaren sind durchlässig für Wasser und alle nicht an Bluteiweiss gebundenen Substanzen bis zu einem Molekulargewicht von 10 000–50 000 Dalton, Blutkörperchen und Bluteiweiss werden zurückgehalten. Bei diesem Verfahren wird die Funktion der natürlichen Nieren nachgeahmt. Die Filtration wird ohne extrakorporale Pumpe aus der Antriebsenergie des menschlichen Kreislaufs betrieben. Zur Aufrechterhaltung dieses extrakorporalen Kreislaufs ist nur etwa 1–2% der Pumpleistung des Herzens notwendig. Da der Druckgradient zwischen arteriellem und venösem Zugang die treibende Kraft darstellt, wurde das Verfahren arterio-venöse Hämofiltration genannt. Das gebildete Filtrat wird verworfen und hinter dem Filter durch eine Substitutionslösung ersetzt.

Im Verlauf einer Stunde muss das Pflegepersonal 500–1000 ml Flüssigkeitsaustausch manuell bilanzieren. Die manuelle Bilanzierung eines täglichen Flüssigkeitsaustausches von 12–20 Litern ist ein hoher Aufwand für das Pflegepersonal und ist als mögliche Fehlerquelle nicht ohne Risiko.

Durch die DE-OS 2 629 717 ist zwar eine Vorrichtung gemäss einleitendem Teil des Patentanspruchs 1 zur Steuerung des Flüsssigkeitsgleichs bei einer Hämodiafiltration bekannt geworden, womit eine manuelle Bilanzierung umgangen werden kann. Das Gerät, das einen Waagenteil enthält, arbeitet jedoch nur mit Fremdenergie. Es sind elektrische Pumpen für das Blut, für das Filtrat und die Ersatzlösung vorgesehen. Auch der Waagenteil arteitet mit einem motorisch verstellbaren Schiebgewicht. Zusätzlich ist das Gerät durch elektrische Regelunsvorrichtungen kompliziert gestaltet, gross, netzabhängig und teuer. Ausserdem kann es nur zusammen mit einem Dialysegerät verwendet werden.

Der Erfindung liegt die Aufgabe zugrunde, eine einfache Vorrichtung ohne Einsatz von Fremdenergie zu schaffen, mit der eine Bilanzierung des Flüssigkeitsaustausches bei der spontanen arterio-venösen Hämofiltration kontinuierlich möglich ist, damit die medizinischen und ökonomischen Nachteile konventioneller Therapien wie der Hämodialyse vermieden werden können.

Insbesondere soll eine derartige Vorrichtung bedien- und kontrollierbar sein, ohne dass an das Pflegepersonal spezielle Ausbildungsanforderungen gestellt werden. Die Vorrichtung soll in der Lage sein, selbsttätig den Flüssigkeitsaustausch zu bilanzieren, so dass das Risiko, das mit einer Bilanzierung von Hand verbunden ist, ausscheidet.

Die erfindungsgemässe Lösung besteht in einer Vorrichtung gemäss Patentanspruch 1 mit einer mechanischen Waage, deren Lastseite durch die Summe der Massen des Filtrats und der Substitutionslösung belastbar ist, wobei zur Ausnutzung der Schwerkraft als Strömungsantrieb für den Zufluss der Substitutionslösung zum Patienten wie auch für den Abfluss des Filtrats vom Patienten und vom Filter der Substitutionslösungsbehälter oberhalb des Filtratbehälters angeordnet und ein mechanisch mit der Waage verbundenes Drossel- und Absperrorgan zur Steuerung der Zufuhr der Substitutionslösung vorgesehen ist. In einer Ausführungsart wird die am oberen Waagebalken in 2 Metern Höhe angehängte Ersatzlösung (4,5 Liter Beutel) von einem Gegengewicht auf der gegenüberliegenden Hebelseite austariert. Wenn Hämofiltrat in den Auffangbehälter tropft, der am unteren Hebelarm unterhalb der Substitutionslösung aufgehängt ist, öffnet das zunehmende Ungleichgewicht mechanisch eine Schlauchklemme und passt die Substitutionsrate mit hoher Genauigkeit ($\pm$ 10 ml/4,5 Liter Austausch) an die Filtrationsrate an. Der untere Hebelarm ist in 76 cm Höhe angebracht, der Filtratbehälter (10 Liter Kapazität) hängt in einer Höhe von 42 cm mit der oberen Öffnung. Bei dieser Anordnung ist es möglich, einen zusätzlichen Unterdruck auf der Filtratseite aufzubauen zwischen der Höhe des Hämofilters (Patientenbett) und dem Ende den Filtratschlauchs unmittelbar über dem Filtratbehälter.

Der obere Waagebalken wurde in 2 Metern Höhe angebracht, um einen ausreichenden hydrostatischen Druck zu erzielen, der selbst dann ausreicht, wenn durch ein zusätzliches Filtersystem substituiert wird. Die Schlauchklemme ist aufgebaut aus einem Schlauchbett, in das ein elastisches Schlauchsegment (Siliconschlauch integriert in die Substitutionsschlauchleitung) eingespannt wird. Ein beweglicher Klemmstift ist mechanisch an den Verbindungsstab der Waagenhebel gekoppelt.

Zur genauen Justierung des Systems, besonders im Hinblick auf geringe Unterschiede des Beutelgewichts bei verschiedenen Herstellern, kann der genaue Einsatzpunkt der Regelung durch die Schlauchklemme so verändert werden, dass ein zusätzliches Gegengewicht von 650 g verschieblich auf einer Gewindestange in der Verlängerung des unteren Hebelarms angebracht ist.

Um eine Negativbilanz zu ermöglichen, kann eine zusätzliche Vorrichtung angebracht sein. Auf der Gegengewichtsseite ist dabei ein graduierter Behälter aufgehängt, der durch eine Tropfinfusion gefüllt werden kann. Die Tropfinfusionsrate bestimmt das Ausmass der Negativbilanz. Zum Beispiel ist der Filtratbehälter am Ende eines Austausches mit 4,5 Liter und z.B. 2 Liter zusätzl. Filtrat gefüllt, wenn 2 Liter Negativbilanz erwünscht sind.

Mit der erfindungsgemässen Vorrichtung steht der spontanen kontinuierlichen Hämofiltration für Intensivpflegepatienten ein in der Einfachheit seines technischen Aufbaus kaum zu übertreffendes Gerät für die automatische Bilanzierung zur Verfügung. Ein bisher nicht gelöstes Problem bei der kontinuierlichen arterio-venösen Hämofiltration war die manuell notwendige Bilanzierung von 12–20 Litern Austauschflüssigkeit täglich. Mit dem beschriebenen System ist ein einmaliger Wechsel von Substitutionsbeutel und Filtratbehälter pro Pflegeschicht notwendig (3mal/24h). Die Sicherheit des Systems ist mindestens so hoch wie die einer einfachen Tropfindusion. Mit einer kurzen Einweisung ist es möglich, das Pflegepersonal jeder Intensivpflegestation mit dem System problemlos vertraut zu machen.

Risiken, die mit der üblichen manuellen Bilanzüberwachung verbunden sind, sind nunmehr vermeidbar. Die Kosten des Systems, selbst bei simultaner Anwendung mehrerer Systeme können erheblich gesenkt werden im Vergleich zu konventionellen Behandlungsverfahren (Hämodialyse und maschinelle Hämofiltration). Darüberhinaus kann das System auch für verschiedene andere Flüssigkeitsbilanzierungen angewendet werden, wie forcierte Diurese zur Behandlung von Intoxikationen, Darmspülungen, arterio-venösen Plasmaaustauch und mit geringen Modifikationen auch für die kontinuierlich zyklische Periotonealdialyse. Auch für chemische oder technische Verfahrenstechniken könnte das System benutzt werden.

In der Zeichnung sind rein schematisch Ausführungsbeispiele erläutert. Es zeigen:

Figur 1: die Gesamtansicht der Vorrichtung als Hebelwaage;

Figur 2: ein Detail im Bereich der Schlauchklemme der Vorrichtung gemäss Figur 1;

Figur 3: die Ausbildung der Vorrichtung als Federwaage bzw. als pneumatische Waage.

An einem senkrechten Ständer 1 mit einem Standfuss 2 sind zwei mit Abstand voneinander angeordnete Hebel 3 und 4 drehbar gelagert. Die Hebellager sind mit 5 und 6 bezeichnet. Die gewichtsseitigen Enden 3b und 4b sind mit Gelenken 8 und 9 ausgerüstet, zwischen welchen sich eine Verbindungsstange 7 erstreckt. Der Hebel 3 dient an seinem lastseitigen Ende 3a zur Aufnahme eines Behälters 10 für eine dem Patienten zuzuführende Substitutionslösung, während an dem lastseitigen Hebelende 4a des Hebels 4 ein graduierter Auffangbehälter 11 für ausgeschiedenes Filtrat befestigt ist. Von dem Behälter 10 der Substitutionslösung führt eine Schlauchleitung 12 mit einer Tropfkammer 14 zum Patienten. Die vom Hämofilter kommende Schlauchleitung 13 hängt in der Höhe verstellbar über einer grossen Öffnung des Filtratbehälters 11.

In der oberen Hälfte des Ständers ist die Schlauchklemme montiert. Sie besteht aus einem Schlauchbett (Klemmtisch), in das ein Stück Siliconschlauch mit hoher Rückstellelastizität eingespannt wird. Das Siliconschlauchstück ist in die Substitutionsschlauchleitung 12 integriert.

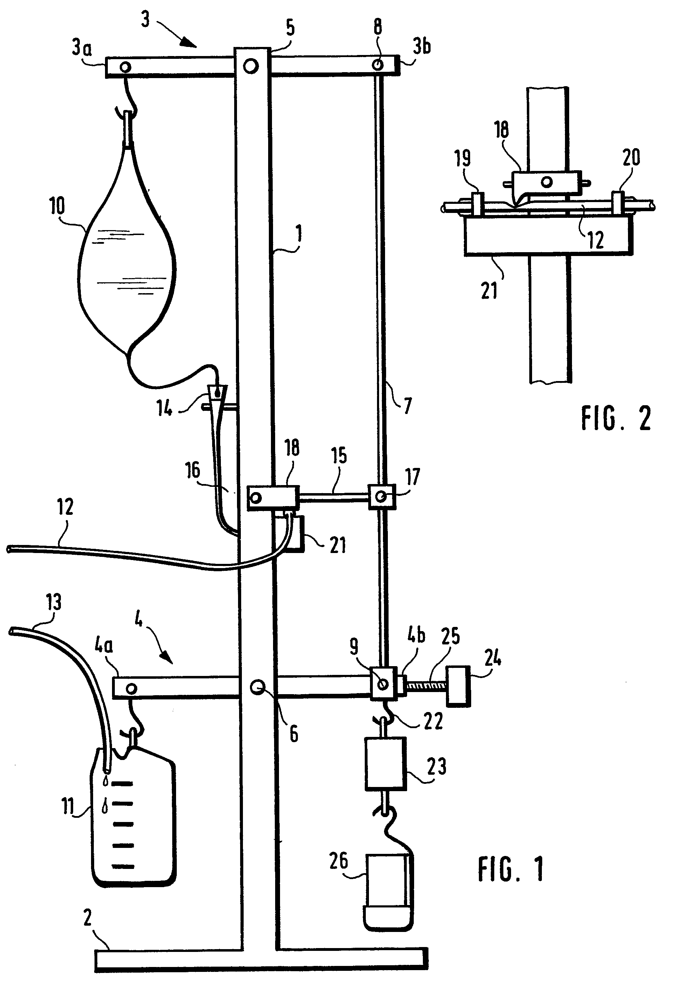

Das Schlauchbett 21 kann horizontal (wie in Abb. 2) oder vertikal angebracht werden. Ein einarmiger Hebel 15 ist über ein Gelenk 17 mit einem Ende an der Verbindungsstange gelagert und mit dem anderen Ende an der Klemmbacke 18.

Um die auf den Schlauch einwirkenden Kräfte zu begrenzen, ist der einarmige Hebel entweder mit einem Zwischenstück aus Federstahl versehen oder es wird eine andere Anordnung, die nicht in der Abbildung gezeigt ist, gewählt. Diese besteht aus einem vertikal montierten Schlauchbett, einem horizontal bewegten Klemmstift, der von einem vertikalen Arm der Klemmbacke bewegt wird. Am Lagerungspunkt ist der bewegliche vertikale Arm der Klemmbacke über eine Torsionsfeder, die in einer Teflonkammer sitzt an die eigentliche Klemmbacke gekoppelt. Die Lagerung ist für die Klammfunktion optimiert.

Am unteren Hebel 4 befindet sich ein Haken 22 zur Aufnahme des Kontrollgewichts 23 der Waage als Gegengewicht zur Masse der Substitutionslösung 10.

Zur genauen Einjustierung des Einsatzpunktes der Schlauchklemme dient ein Justiergewicht 24, das mit Hilfe eines Schraubgewindes 25 in Längsrichtung des Hebels 4 bewegbar ist. Dadurch ist es möglich, das Hebeldrehmoment genau auf den Einsatzpunkt zu justieren, bei dem die Substitionsrate exakt der Filtrationsrate entspricht.

An das Kontrollgewicht 23 kann ein graduierter Behälter 26 angehängt werden. Mit dieser Anordnung ist es auf einfache Weise möglich, eine Negativbilanzierung des Flüssigkeitsaustausches zu erreichen.

Wenn das System unter klinischen Bedingungen eingesetzt wird, nach Anschluss der kontinuierlich arterio-venösen Hämofiltration, wird der filtratführende Schlauch 13 geöffnet und über der Öffnung des Filtratbehälters 11 frei fixiert. Das Justiergewicht wird dann in die Regelposition der Schlauchklemme plaziert.

Der Regelpunkt der Schlauchklemme wird dann mit Hilfe des Justiergewichts 24 eingestellt. Wenn bei überwässerten Patienten ein akuter Gewichtsentzug notwendig ist, wird der Behälter für die Negativbilanz 26 gefüllt bis zur Höhe des notwendigen Entzugs. In diesem Fall bleibt die Schlauchklemme geschlossen, bis das Filtrat exakt der Flüssigkeitsmenge im Behälter 26 entspricht. Danach arbeitet das System wieder im Gleichgewichtszustand und die Massenpunkte verschieben sich langsam vom oberen zum unteren Hebel.

Die Vorrichtung kann auch mit einem einzelnen Waagebalken konstruiert werden, an dessen Lastseite die Substitutionslösung und an einem Überbrückungsrahmen die Haltestange für den Auffangbehälter montiert ist.

Wenn eine kontinuierliche Negativbilanz notwendig ist, wird eine Tropfinfusion am Ständer angebracht (nicht abgebildet) und in den Behälter für die Negativbilanz geleitet 26. Die Geschwindigkeit der Tropfinfusion bestimmt dann die Negativbilanz.

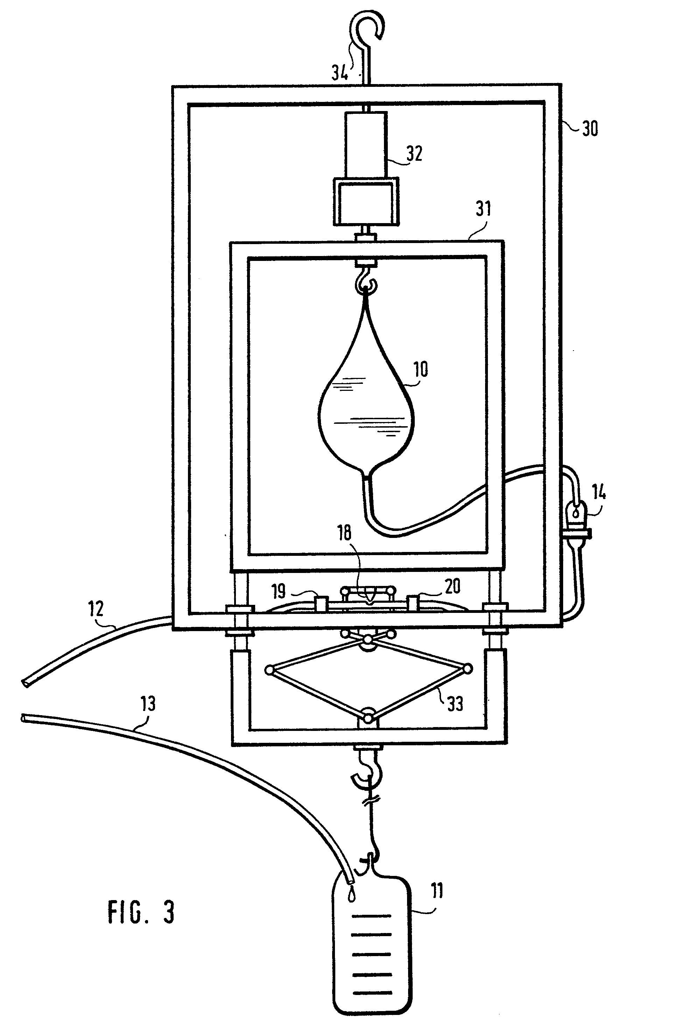

Figur 3 zeigt schematisch die Vorrichtung, aufgebaut als hängender Doppelrahmen 30. 31, bei dem das Gegengewicht ersetzt wurde durch eine Federwaage 32 oder einen pneumatischen Zylinder zwischen den beiden Rahmen und bei dem der Substitutionslösungsbeutel 10 und der Filtratbehälter am inneren Raumen 31 hängen. Die Bewegung der beiden Rahmen gegeneinander kann dann zur Regelung der Schlauchklemme 18 ausgenutzt werden. Zur Übertragung der Verschiebungsstrecken auf die Schlauchklemme 18 ist eine Scherenhebelanordnung 33 zwischen den Rahmen vorgesehen. Sobald das Summengewicht (von Substitutionslösung und Filtrat) ansteigt, öffnet die Schlauchklemme und passt die Substitionsrate der Filtrationsrate an. Auch bei dieser Anordnung kann eine Vorrichtung für eine Negativbilanz über Umlenkrollen angebracht werden, die den inneren Rahmen durch das Zusatzgewicht des Behälters für die Negativbilanz anhebt (nicht abgebildet). Das System kann an einem Deckenhaken 34 aufgehängt werden.

## Patentansprüche

1. Vorrichtung zur kontinuierlichen Bilanzierung von Filtrat und Substitutionslösung bei therapeutischen Verfahren zur Eliminierung von Schadstofen aus dem menschlichen Organismus, mit einer mechanischen Waage (1, 3, 4, 7), deren Lastseite durch die Summe der Massen des Filtrats (11) und der Substitutionslösung (10) belastbar ist und mit einem mechanisch mit der Waage verbundenen Drossel- und Absperrorgan (18) zur Steuerung der Zufuhr der Substitutionslösung, dadurch gekennzeichnet, dass zur Ausnutzung der Schwerkraft als Strömungsantrieb für den Zufluss (12) der Substitutionslösung zum Patienten wie auch für den Abfluss (13) des Filtrats vom Patienten und vom Filter insbesondere zur Bilanzierung bei spontanen arterio-venösen Blutfiltrationen der Substitutionslösungsbehälter (10) oberhalb des Filtratbehälters (11) angeordnet ist.

2. Vorrichtung nach Anspruch 1, dadurch gekennzeichnet, dass die Waage als Hebelwaage (1, 3, 4, 7) ausgebildet und das Drossel- und Absperrorgan (18) mechanisch mit dem Waagenhebel (3, 4) verbunden ist.

3. Vorrichtung nach Anspruch 1, dadurch gekennzeichnet, dass die Waage als Federwaage (30, 31, 32, 33) ausgebildet ist und aus zwei ineinander angeordneten und gegeneinander verschiebbaren Rahmen (30, 31) besteht, von welchen der äussere (30) ortsfest aufhängbar und der innere (31) zur Aufnahme der Gewichtskräfte der Substitutionslösung (10) und des Filtrats (11) vorgesehen ist, wobei zwischen den Rahmen das die Gewichte der Massen bestimmende Federglied (32) befestigt und eine Hebeleinrichtung (33) zur Betätigung des Drossel- und Absperrorgans vorgesehen ist.

4. Vorrichtung nach Anspruch 2, dadurch gekennzeichnet, dass der Waagenhebel (3, 4) in einen Lasthebel (3) für die Masse der Substitutionslösung (10) und in einen hierzu parallelen, auf tieferem Niveau angeordneten Lasthebel (4) für die Filtratmasse (11) unterteilt ist und beide Lasthebel durch einen Verbindungsstab (7) gelenkig miteinander verbunden sind.

5. Vorrichtung nach Anspruch 4, dadurch gekennzeichnet, dass beide Hebel (3, 4) in einem gemeinsamen Ständer (1) gelagert sind.

6. Vorrichtung nach einem der Ansprüche 2, 4 und 5, dadurch gekennzeichnet, dass das Drossel- und Absperrorgan als Schlauchklemme (18) ausgebildet ist und über einen mit dem Waagenhebel (3, 4) verbundenen Klemmhebel (15) betätigbar ist.

7. Vorrichtung nach Ansprüchen 4 und 6, dadurch gekennzeichnet, dass der Klemmhebel (15) als einarmiger, am Ständer (1) gelagerter Hebel ausgebildet ist, dessen Kraftangriffsende über ein Gelenk (17) am Verbindungsstab (7) der beiden Lasthebel (3, 4) befestigt ist.

8. Vorrichtung nach einem der Ansprüche 2 und 4 bis 7, dadurch gekennzeichnet, dass die Armlänge des Waagenhebels (3, 4) am gewichtsseitigen Ende durch ein ein- und ausschraubbares Endstück (24, 25) veränderbar ist.

9. Vorrichtung nach einem oder mehreren der Ansprüche 1, 2, 4 bis 9, dadurch gekennzeichnet, dass am Kontrollgewicht (23) der Waage ein Behälter (26) zur Negativbilanzierung befestigt ist.

10. Vorrichtung nach einem der Ansprüche 1 bis 9, dadurch gekennzeichnet, dass am Ständer (1) ein Tropfinfusionsbehälter befestigbar ist, dessen Ableitung in den Behälter (26) für die Negativbilanzierung führt.

11. Vorrichtung nach einem der Ansprüche 1 bis 10, dadurch gekennzeichnet, dass die Ablaufhöhe des Filtratschlauches (13) zur Beeinflussung der Filtrationsrate veränderbar ist.

12. Vorrichtung nach einem der Ansprüche 1 bis 11, dadurch gekennzeichnet, dass das Drossel- und Absperrorgan als Schlauchklemme (18) mit einer Scherenhebelanordnung (33) ausgebildet ist.

## Claims

1. Device for continuous balancing of filtrate and substitution solution in therapeutic methods for the elimination of harmful substances from the human organism, having a mechanical balance (1, 3, 4, 7), the load side of which can be loaded by the total of the masses of the filtrate (11) and of the substitution solution (10), and a restriction and shut-off element (18), mechanically connected to the balance, for controlling the feed of substitution solution, characterized in that, for utilizing gravity as the driving force for the feed flow (12) of substitution solution to the patient and also for the outflow (13) of the filtrate from the patient and from the filter, in particular for balancing in the case of spontaneous arteriovenous blood filtrations, the substitution solution vessel (10) is arranged above the filtrate vessel (11).

2. Device according to Claim 1, characterized in that the balance is designed as a lever balance (1, 3, 4, 7) and the restriction and shut-off element (18) is mechanically connected to the balance arm (3, 4).

3. Device according to Claim 1, characterized in that the balance is designed as a spring balance (30, 31, 32, 33) and consists of two frames (30, 31) which are arranged within one another and are displaceable relative to one another and of which the outer frame (30) can be suspended in a fixed position and the inner frame (31) is provided for taking up the weight forces of the substitution solution (10) and of the filtrate (11), the spring member (32) which determines the weights of the masses being fixed between the frames, and a lever device (33) being provided for actuating the restriction and shut-off element.

4. Device according to Claim 2, characterized in that the balance arm (3, 4) is devided into a load arm (3) for the mass of the substitution solution (10) and a load arm (4), parallel thereto and arranged at a lower level, for the filtrate mass (11), and the two load arms are articulated to one another by a connecting rod (7).

5. Device according to Claim 4, characterized in that the two arms (3, 4) are mounted in a common stand (1).

6. Device according to one of Claims 2, 4 and 5, characterized in that the restriction and shut-off element is in the form of a hose clip (18) and can be actuated via a clamping lever (15) connected to the balance arm (3, 4).

7. Device according to Claims 4 and 6, characterized in that the clamping lever (15) is in the form of a singlearm lever which is mounted on the stand (1) and the forceapplying end of which is fixed via a hinge (17) to the connecting rod (7) of the two load arms (3, 4).

8. Device according to one of Claims 2 and 4 to 7, characterized in that the arm length of the balance arm (3, 4) on the weight-side end is variable by means of an end piece (24, 25) which can be screwed in and out.

9. Device according to one or more of Claims 1, 2 and 4 to 9, characterized in that a vessel (26) for negative balancing is fixed to the control weight (23) of the balance.

10. Device according to one of Claims 1 to 9, characterized in that a drip feed vessel, the outflow line of which leads into the vessel (26) for negative balancing, can be fixed to the stand (1).

11. Device according to one of Claims 1 to 10, characterized in that the outflow height of the filtrate hose (13) is variable for adjusting the filtration rate.

12. Device according to one of Claims 1 to 11, characterized in that the restriction and shut-off element is in the form of a hose clip (18) with a scissors-type lever arrangement (33).

## Revendications

1. Dispositif pour l'équilibrage en continu du filtrat et de la solution de substitution dans les méthodes thérapeutiques pour l'élimination de substances nocives de l'organisme humain, comprenant une balance mécanique (1, 3, 4, 7) dont on peut charger le côté de charge à l'aide du total des masses du filtrat (11) et de la solution de substitution (10), et un élément de restriction et de coupure (18), relié mécaniquement à la balance pour contrôler l'alimentation en solution de substitution, caractérisé en ce que, dans le but d'utiliser la gravité en tant que force d'entraînement pour l'écoulement d'alimentation (12) de solution de substitution vers le patient et aussi pour l'écoulement vers l'extérieur (13) du filtrat à partir du patient et à partir du filtre, en particulier pour l'équilibrage dans le cas des filtrations de sang artériovéineux spontanées, le récipient de solution de substitution (10) est disposé au-dessus du récipient de filtrat (11).

2. Dispositif selon la revendication 1, caractérisé en ce que la balance est conçue comme une balance à levier (1, 3, 4, 7) et en ce que l'élément de restriction et de coupure (18) est relié, mécaniquement au bras de la balance (3, 4).

3. Dispositif selon la revendication 1, caractérisé en ce que la balance est conçue comme une balance à ressort (30, 32, 32, 33) et en ce qu'elle est constituée de deux cadres (30, 31) qui sont disposés l'un à l'intérieur de l'autre et qui sont mobiles l'un par rapport à l'autre et parmi lesquels on peut suspendre le cadre extérieur (30) en position fixe et en ce que le cadre interne (31) est prévu pour absorber les forces pondérales de la solution de substitution (10) et du filtrat (11), l'élément de ressort (32) qui détermine les poids des masses étant fixé entre les cadres, et un dispositif de levier (33) étant prévu pour actionner l'élément de restriction et de coupure.

4. Dispositif selon la revendication 2, caractérisé en ce que le bras de la balance (3, 4) est divisé en un bras de charge (3) pour la masse de la solution de substitution (10) et un bras de charge (4), parallèle à celui-ci et disposé à un niveau inférieur pour la masse du filtrat (11), et en ce que les deux bras de charge sont articulés l'un à l'autre par une tige de raccordement (7).

5. Dispositif selon la revendication 4, caractérisé en ce que les deux bras (3, 4) sont montés sur un support commun (1).

6. Dispositif selon l'une quelconque des revendications 2, 4 et 5, caractérisé en ce que l'élément de restriction et de coupure se présente sous forme d'une pince à tube souple (18) et en ce qu'il peut être actionné par un levier de pincement (15) relié au bras de la balance (3, 4).

7. Dispositif selon les revendications 4 et 6, caractérisé en ce que le levier de pincement (15) se présente sous la forme df'un levier à bras unique qui est monté sur le support (1) et dont l'extrémité appliquant la force est fixée par une charnière (17) à la tige de raccordement (7) des deux deux bras de charge (3, 4).

8. Dispositif selon l'une quelconque des revendications 2 et 4 à 7, caractérisé en ce que l'on peut faire varier la longueur du bras de la balance (3, 4) à l'extrémité du côté du poids au moyen d'une pièce d'extrémité (24, 25) que l'on peut visser et dévisser.

9. Dispositif selon l'une ou plusieurs des revendications 1, 2 et 4 à 9, caractérisé en ce qu'un récipient (26) pour l'équilibrage négatif est fixé au poids de contrôle (23) de la balance.

10. Dispositif selon l'une quelconque des revendications 1 à 9, caractérisé en ce qu'on peut fixer au support (1) un récipient d'alimentation pour goutte à goutte, dont la ligne d'écoulement de sortie mène dans le récipient (26) pour l'équilibrage négatif.

11. Dispositif selon l'une quelconque des revendications 1 à 10, caractérisé en ce que l'on peut faire varier la hauteur d'écoulement de sortie du tube souple de filtrat (13) pour ajuster la vitesse de filtration.

12. Dispositif selon l'une quelconque des revendications 1 à 11, caractérisé en ce que l'élément de restriction et de coupure se présente sous forme d'une pince à tube souple (18) et est disposé comme un levier de type ciseaux (33).

0 093 259

FIG. 2

FIG. 1

7

FIG. 3